(19) Europäisches Patentamt
European Patent Office
Office européen des brevets

(11) **EP 0 650 718 B1**

(12) **EUROPÄISCHE PATENTSCHRIFT**

(45) Veröffentlichungstag und Bekanntmachung des Hinweises auf die Patenterteilung:
24.03.1999 Patentblatt 1999/12

(51) Int. Cl.⁶: **A61K 7/13**

(21) Anmeldenummer: 94110706.2

(22) Anmeldetag: 09.07.1994

(54) **Oxidationshaarfärbemittel aus einer cremeförmigen Farbstoffträgermasse und einer ein Polymer und ein Oxidationsmittel enthaltenden Zubereitungen sowie Verfahren zum oxidativen Färben von Haaren**

Hair oxidation composition based on a creamy carrier and a preparation constituted by a polymer and an oxydation hair dye and process for an oxidation hair dyeing

Composition de coloration oxydative des cheveux constituée d'un support colorant crèmeux et d'une préparation contenant un polymère et un oxydant et procédé de coloration oxydative des cheveux

(84) Benannte Vertragsstaaten:
**DE ES FR GB IT**

(30) Priorität: 28.09.1993 DE 4332965

(43) Veröffentlichungstag der Anmeldung:
03.05.1995 Patentblatt 1995/18

(73) Patentinhaber:
**Wella Aktiengesellschaft
64274 Darmstadt (DE)**

(72) Erfinder:
• **Mager, Herbert, Dr.
CH-1723 Marly (CH)**
• **Aeby, Johann
CH-1723 Marly (CH)**

(56) Entgegenhaltungen:
**DE-A- 1 492 125        DE-A- 4 018 259
FR-A- 2 569 346**

**Beschreibung**

Gegenstand der Erfindung ist ein Mittel zum oxidativen Färben von Haaren, das durch Vermischen einer cremeförmigen Farbträgermasse mit einer ein nichtionisches Polymer und ein Oxidationsmittel enthaltenden Zubereitung erhalten wird, sowie ein Verfahren zum oxidativen Färben von Haaren.

In der Haarfärbepraxis haben Oxidationshaarfärbemittel eine wesentliche Bedeutung erlangt. Die Färbung erfolgt hierbei im Haarschaft durch die Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 4-Aminophenol, 4-Amino-3-methylphenol, 1,4-Diaminobenzol, 2-(2'-Hydroxyethyl)-1,4-diaminobenzol, 2,5-Diaminobenzylalkohol und Tetraaminopyrimidin verwendet. Bevorzugt eingesetzte Kupplersubstanzen sind 1-Naphthol, Resorcin, 4-Chlorresorcin, Sesamol, m-Aminophenol, 5-Amino-o-kresol, 2-Amino-4-(2'-hydroxyethyl)aminoanisol, 2,4-Diaminoanisol und 2,4-Diaminophenetol.

Durch geeignete Kombinationen von Entwicklersubstanzen und Kupplersubstanzen läßt sich eine breite Palette verschiedener Farbnunancen erzeugen.

Oxidationshaarfärbemittel bestehen aus zwei Komponenten, die kurz vor dem Gebrauch vermischt und dann auf das zu färbende Haar aufgebracht werden. Die erste Komponente, die Farbträgermasse, enthält die färberisch wirksamen Substanzen und kann in Form einer Lösung, eines Gels oder als Creme vorliegen. Die zweite Komponente ist ein Produkt in dem ein geeignetes Oxidationsmittel, zum Beispiel Wasserstoffperoxid, enthalten ist.

In der Färbepraxis werden vorzugsweise Farbträgermassen in Form einer hochviskosen Creme mit einer flüssigen oder emulsionsförmigen wäßrigen Wasserstoffperoxidlösung vermischt und die so erhaltenen cremeförmigen Zubereitungen mit einem Pinsel auf die zu färbenden Haare aufgetragen. Die Verwendung von dickflüssigen Färbecremes wird insbesondere dann bevorzugt, wenn bei stark ergrautem Haar eine vollständige Grauabdeckung sowie ein ausgezeichneter Farbausgleich erzielt werden soll, da eine hochviskose Creme mit einem Pinsel gezielter und mit einer höheren Schichtdicke auf das Haar aufgetragen werden kann als eine dünnflüssige Zubereitung, die dazu neigt, vom Haar abzulaufen.

Derartige hochviskose Färbecremes besitzen jedoch neben diesen Vorteilen eine Reihe von Nachteilen. So läßt sich die hochviskose Farbträgermasse mit der flüssigen Wasserstoffperoxidlösung nur schwer zu einem einheitlichen Färbemittel vermischen. Weiterhin ist die Applikation mit einer Auftrageflasche aufgrund der hohen Viskosität und des schlechten Fließverhaltens nicht möglich.

Aus diesem Grunde werden in der Praxis hochviskose Färbecremes und speziell für die Verwendung in Auftrageflaschen geeignete niedrigviskose Zubereitungen nebeneinander eingesetzt.

Hierdurch wird es erforderlich, verschiedene Formulierungen zu entwickeln, die entweder für das Auftragen mit einem Pinsel oder für das Auftragen mit einer Auftrageflasche geeignet sind.

Dies ist nicht nur mit höheren Entwicklungskosten verbunden, sondern führt auch zu einer überflüssigen Produkt- und Rohstoffvielfalt, wodurch unter anderem hohe Lagerkosten sowohl beim Hersteller als auch beim Anwender entstehen.

Es bestand daher die Aufgabe, durch eine geeignete Modifikation der überlicherweise mit einem Pinsel aufzutragenden hochviskosen Cremehaarfarben eine Verwendung dieser Cremehaarfarben in Auftrageflaschen zu ermöglichen.

Aus der DE-A-40 18 259 sind Wasserstoffperoxidzubereitungen bekannt, welche als Schaumregulator bestimmte nichtionische Tenside der Formel $R^1$-O$(C_2H_4O)_x$-$R^2$ enthalten und zur Verwendung mit einer Haarfärbe- oder Blondiercreme bestimmt sind.

Es wurde nunmehr überraschenderweise gefunden, daß eine übliche hochviskose, cremeförmige Farbträgermasse bei Verwendung einer ein nichtionisches Polymer enthaltenden Oxidationsmittelzubereitung auch für die Anwendung mittels einer Auftrageflasche verwendbar ist.

Gegenstand der vorliegenden Erfindung ist daher ein Mittel zum oxidativen Färben von Haaren, welches durch Vermischen einer hochviskosen, cremeförmigen Farbträgermasse (A) mit einer flüssigen oder emulsionsförmigen, ein Oxidationsmittel enthaltenden Zubereitung (B) erhalten wird, und dadurch gekennzeichnet ist, daß die das Oxidationsmittel enthaltende Zubereitung (B) ein nichtionisches Polymer der Formel (I)

$$R\text{-}(O\text{-}CH(CH_3)\text{-}CH_2)_x\text{-}(O\text{-}CH_2\text{-}CH_2)_y\text{-}OH \qquad (I),$$

mit

R = $C_1$-bis $C_{25}$-Alkyl, $C_2$-bis $C_{25}$-Hydroxyalkyl oder $C_3$-bis $C_{25}$-Dihydroxyalkyl;
x = einer ganzen Zahl von 0 bis 200;
y = einer ganzen Zahl von 0 bis 200 und
x + y $\geq$ 20 ,

enthält.

Das erfindungsgemäße Oxidationshaarfärbemittel ermöglicht eine ausgezeichnete Haarfärbung sowie eine gute Abdeckung von ergrautem Haar und ist aufgrund seiner vergleichsweise niedrigen Viskosität, seines ausgezeichneten Fließverhaltens, sowie der guten Mischbarkeit der Komponenten (A) und (B) hervorragend für die Anwendung mittels einer Auftrageflasche geeignet.

In einer bevorzugten Ausführungsform des erfindungsgemäßen Mittels beträgt das Gewichtsverhältnis der Färbemasse (A) zur Zubereitung (B) 2:1 bis 1:3, wobei ein Gewichtsverhältnis von 1:1 besonders bevorzugt ist.

Die Viskosität des gebrauchsfertigen Mittels zum oxidativen Färben von Haaren liegt vorzugsweise in einem Bereich von 500 bis 3000 mPa.s, wobei die Viskosität der Komponente (A) vorzugsweise mindestens 10.000 mPa.s betragen soll, während die Komponente (B) vorzugsweise eine Viskosität von maximal 2000 mPa.s aufweisen soll.

Als nichtionisches Polymer der Formel (I) sind beispielsweise die im CTFA-International Cosmetic Ingredient Dictionary, 4. Auflage (1991) auf den Seiten 475 bis 490 beschriebenen Verbindungen geeignet, wobei der Polyoxyethylen(10)polyoxypropylen(7)butylether, der Polyoxyethylen(30)polyoxypropylen(20)butylether, der Polyoxyethylen(20)polyoxypropylen(10)cetyl/stearylether, der Polyoxyethylen(20)polyoxypropylen(5)cetylether, der Polyoxyethylen(10)polyoxypropylen(20)decyltetradecylether, der Polyoxyethylen(24)polyoxypropylen(24)glycerylether, der Polyoxyethylen(50)polyoxypropylen(12)lanolinether, der Polyoxyethylen(12)polyoxypropylen(66)glycerylether und insbesondere der Polyoxyethylen(45)polyoxypropylen(33)butylether und der Polyoxyethylen(37)polyoxypropylen(38)butylether bevorzugt sind.

Das nichtionische Polymer der Formel (I) wird in der Zubereitung (B) in einer Menge von 0,01 bis 6 Gewichtsprozent eingesetzt.

Die Zubereitung (B) enthält neben dem Polymer der Formel (I) 0,1 bis 20 Gewichtsprozent, vorzugsweise 2 bis 14 Gewichtsprozent, eines Oxidationsmittels. Als Oxidationsmittel zur Entwicklung der Haarfarbe kommen insbesondere Wasserstoffperoxid sowie dessen Additionsverbindungen an Harnstoff, Melamin und Natriumborat in Betracht, wobei Wasserstoffperoxid besonders bevorzugt ist.

Weiterhin kann die Zubereitung (B) für derartige Zubereitungen übliche Stoffe, wie zum Beispiel Verdicker, insbesondere Fettalkohole, Fettsäureester und Vaseline; Emulgatoren, beispielsweise Natriumlaurylalkoholdiglykolethersulfat und Cholesterin; Parfümöle; Stabilisatoren, beispielsweise Salicylsäure und p-Hydroxybenzoesäureester; anorganische Säuren, insbesondere Phosphorsäure; sowie Komplexbildner und Trübungsmittel enthalten.

Die hochviskose Farbträgermasse (A) enthält 15 bis 60 Gewichtsprozent eines Verdickergemisches, welches vorzugsweise zu mindestens 50 Gewichtsprozent aus $C_{10}$-bis $C_{24}$-Fettalkoholen, vorzugsweise Cetylalkohol und Stearylalkohol oder deren Gemischen, besteht. Neben diesen Fettalkoholen kann das Verdickergemisch weitere für kosmetische Zubereitungen gebräuchliche Verdicker, wie zum Beispiel Fettsäureester; Vaseline; mit 2 bis 6 Mol Ethylenoxid oxethylierte Fettalkohole; mit 2 bis 8 Mol Ethylenoxid oxethylierte Nonylphenole; Fettsäuren, beispielsweise Ölsäure; Stärke; Walrat oder verdickend wirkende Polyacrylsäurederivate, enthalten.

Weiterhin kann die Farbträgermasse (A) nichtionogene, anionische oder amphotere Emulgatoren, beispielsweise Natriumlaurylethersulfat, Natriumlaurylsulfat, Ammoniumlaurylsulfat, Kaliumstearat, Cetylalkoholpolyethylenglykolether, oxethyliertes Rizinusöl, Kokosfettsäureamidopropylbetain und Kokosalkyldimethylammoniumbetain; Cholesterin; Wollwachsalkohole; Antioxidantien, zum Beispiel Ascorbinsäure oder Natriumsulfit; Komplexbildner; Parfümöle; Lösungsmittel, beispielsweise Ethanol, Isopropanol, 1,3-Butandiol, Propylenglykol und Glycerin; oder kationische Polymere enthalten.

Die vorgenannten Zusatzstoffe sind in der Farbträgermasse (A) in den für derartige Zubereitungen üblichen Mengen enthalten, zum Beispiel die Lösungsmittel in einer Menge von 1 bis 10 Gewichtsprozent, die Parfümöle in einer Menge von 0,01 bis 1 Gewichtsprozent, die kationischen Polymere in einer Menge von 0,05 bis 1 Gewichtsprozent sowie die Antioxidantien und Komplexbildner in einer Menge von 0,01 bis 0,5 Gewichtsprozent.

Die Farbträgermasse (A) weist einen pH-Wert von 3,5 bis 12,5 auf. Der pH-Wert wird vorzugsweise mit Ammoniak eingestellt. Es können jedoch auch organische Amine, beispielsweise Monoethanolamin, oder anorganische Alkalien wie Natronlauge zur Einstellung des pH-Wertes verwendet werden.

Die Farbträgermasse (A) enthält mindestens eine Kupplersubstanz und mindestens eine Entwicklersubstanz sowie gegebenenfalls zusätzlich mit sich selbst kuppelnde Farbstoffvorstufen und direkt auf das Haar aufziehende Farbstoffe. Die Entwickler- und Kupplersubstanzen werden in dem Haarfärbemittel entweder als solche oder in Form ihrer physiologisch unbedenklichen Salze mit anorganischen oder organischen Säuren, beispielsweise als Chlorid, Sulfat, Phosphat, Acetat, Propionat, Lactat oder Citrat, eingesetzt.

Die Kupplersubstanzen werden im allgemeinen in etwa äquimolarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanzen in einem gewissen Überschuß oder Unterschuß zum Einsatz kommen. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch von bekannten Kupplersubstanzen darstellen.

Die Farbträgermasse (A) enthält als bekannte Kupplersubstanzen, allein oder im Gemisch miteinander, insbesondere 1-Naphthol, 4-Methoxy-1-naphthol, Rescorcin, 4-Chlorresorcin, 4,6-Dichlorresorcin, 2-Methylresorcin, 3-Aminophenol, 3-Amino-6-methylphenol, 4-Hydroxy-1,2-methylendioxybenzol, 4-Amino-1,2-methylendioxybenzol, 4-(2'-Hydroxyethylamino)-1,2-methylendioxybenzol, 4-Hydroxyindol, 2,3-Diamino-6-methoxy-pyridin und 5-Amino-2-methylphenol. Weitere geeignete Kupplersubstanzen sind zum Beispiel 2,4-Dihydroxyphenolether wie 2,4-Dihydroxyanisol und 2,4-Dihydroxyphenoxyethanol.

Von den bekannten Entwicklersubstanzen kommen als Bestandteil der erfindungsgemäßen Farbträgermasse vor allem 1,4-Diaminobenzol, 2,5-Diaminotoluol, 2,5-Diaminobenzylalkohol, 3-Methyl-4-aminophenol, 2-(2'-Hydroxyethyl)-1,4-diaminobenzol, Tetraaminopyrimidin und 4-Aminophenol in Betracht.

Zur Haarfärbung bekannte und übliche Oxidationsfarbstoffe, die in der Farbträgermasse (A) enthalten sein können, sind unter anderem in dem Buch von E. Sagarin, "Cosmetics, Science and Technology", Interscience Publishers Inc., New York (1957), Seiten 503 ff. sowie in dem Buch von H. Janistyn, "Handbuch der Kosmetika und Riechstoffe" (1973), Seiten 388 ff. beschrieben. Die Gesamtmenge der in der Farbträgermasse (A) enthaltenen Entwicklersubstanz-Kupplersubstanz-Kombination soll 0,01 bis 12 Gewichtsprozent, insbesondere 0,2 bis 4 Gewichtsprozent, betragen.

Zur Erzielung gewisser Farbnunancen können ferner auch übliche direktziehenden Farbstoffe, beispielsweise Triphenylmethanfarbstoffe wie Basic Violet 14 (C.I. 42 510) und Basic Violet 2 (C.I. 42 520), aromatische Nitrofarbstoffe wie 2-Amino-4,6-dinitrophenol, 2-Nitro-4-(2'-hydroxyethylamino)-anilin und 2-Amino-4-nitrophenol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue (C.I. 64 500), Disperse Red 15 (C.I. 60 710), Disperse Violet 1 (C.I. 61 100), außerdem 1,4,5,8-Tetraaminoanthrachinon und 1,4-Diaminoanthrachinon, in der Farbträgermasse (A) enthalten sein.

Die Farbträgermasse (A) kann weiterhin auch mit sich selbst kuppelnde Farbstoffvorstufen, wie zum Beispiel 2-Amino-5-methylphenol, 2-Amino-6-methylphenol, 2-Amino-5-ethoxyphenol oder auch 2-Propyl-amino-5-aminopyridin, enthalten.

Die Gesamtmenge der direktziehenden Farbstoffe und der mit sich selbst kuppelnden Farbstoffvorstufen beträgt in der Farbträgermasse (A) 0,01 bis 6 Gewichtsprozent, vorzugsweise 0,2 bis 4 Gewichtsprozent.

Die Gesamtmenge aller Farbstoffe, also der Entwicklersubstanz-Kupplersubstanz-Kombination, der mit sich selbst kuppelnden Farbstoffvorstufen und der direktziehenden Farbstoffe, in der Farbträgermasse (A) soll 0,1 bis 14 Gewichtsprozent, vorzugsweise 0,2 bis 8 Gewichtsprozent, betragen.

Das durch Mischen der Farbträgermasse (A) mit der das Oxidationsmittel enthaltenden Zubereitung (B) entstehende gebrauchsfertige Mittel zum oxidativen Färben von Haaren kann sauer, neutral oder alkalisch eingestellt sein. Der pH-Wert des erfindungsgemäßen Mittels zum oxidativen Färben von Haaren liegt vorzugsweise in einem Bereich von 7,5 bis 12, wobei ein pH-Wert von 9,5 bis 10,5 besonders bevorzugt ist.

Die vorstehenden Gewichtsprozentangaben sind, sofern nichts anderes angegeben ist, jeweils auf die Gesamtmenge der Farbträgermasse (A) beziehungsweise auf die Gesamtmenge der Zubereitung (B) bezogen.

Bei der Anwendung des zuvor beschriebenen Oxidationshaarfärbemittels nach dem erfindungsgemäßen Verfahren vermischt man unmittelbar vor Gebrauch die cremeförmige Farbträgermasse (A) mit der das Oxidationsmittel enthaltenden Zubereitung (B) in einem Gewichtsverhältnis von 2:1 bis 1:3, vorzugsweise 1:1, und trägt eine für die Haarfärbung ausreichende Menge, je nach Haarfülle 90 bis 160 Gramm, des gebrauchsfertigen Oxidationshaarfärbemittels mit Hilfe einer Auftrageflasche auf das Haar auf. Man läßt das Gemisch bei 15 bis 50 Grad Celsius etwa 10 bis 45 Minuten, vorzugsweise 30 Minuten, lang auf das Haar einwirken, spült sodann das Haar mit Wasser aus und trocknet es. Gegebenenfalls wird das Haar vor dem Trocknen gewaschen und/oder mit der Lösung einer physiologisch verträglichen organischen Säure, beispielsweise Zitronensäure oder Weinsäure, nachgespült.

Die nachfolgenden Beispiele sollen den Gegenstand der Erfindung näher erläutern.

**Beispiele**

**Beispiel 1: Oxidationshaarfärbemittel**

| Cremeförmige Farbträgermasse (A1) | |
|---|---|
| 25,000 g | Cetylstearylalkohol |
| 0,600 g | Wollwachsalkohol |

(fortgesetzt)

| Cremeförmige Farbträgermasse (A1) | |
|---|---|
| 0,200 g | Cholesterin |
| 6,800 g | Natriumlaurylalkoholdiglykolethersulfat, 28-prozentige wäßrige Lösung |
| 0,500 g | Natriumsulfit, wasserfrei |
| 1,350 g | 2,5-Diaminotoluolsulfat |
| 0,720 g | Resorcin |
| 0,056 g | m-Aminophenol |
| 0,028 g | m-Phenylendiamin |
| 7,282 g | Ammoniak, 25-prozentige wäßrige Lösung |
| 57,464 g | Wasser |
| $\overline{100,000\ g}$ | |

| Wasserstoffperoxid-Zubereitung (B 1) | |
|---|---|
| 12,0 g | Wasserstoffperoxid, 50-prozentige wäßrige Lösung |
| 2,0 g | Polyoxyethylen(45)polyoxypropylen(33)butylether (Unilube® MB50-168R der Nippon Oil & Fats Co., Ltd.; Tokyo /Japan) |
| 1,0 g | Cetylstearylalkohol |
| 0,2 g | Natriumlaurylsulfat |
| 0,1 g | Polyethylenglykol(25)cetyl/stearylether (Cremophor® A25 der BASF AG; Ludwigshaften/DE) |
| 84,7 g | Wasser |
| $\overline{100,0\ g}$ | |

Der pH-Wert der Wasserstoffperoxid-Zubereitung (B1) wird mit verdünnter Phosphorsäure auf 2,5 eingestellt.

In einer 200 ml-Auftrageflasche werden 50 Gramm der Farbträgermasse (A1) und 50 Gramm der Wasserstoffperoxid-Zubereitung (B1) durch Schütteln miteinander vermischt. Der Schüttelvorgang erfolgt problemlos und ergibt nach kurzem Schütteln eine vollkommen homogene Mischung, die problemlos aus der Auftrageflasche auf das Haar aufgetragen werden kann.

Das so erhaltene Haarfärbemittel wird auf vollständig ergrautes Haar aufgetragen. Nach einer Einwirkungszeit von 30 Minuten bei Raumtemperatur wird das Haarfärbemittel mit Wasser gründlich ausgespült. Anschließend wird das Haar mit einem Shampoo gewaschen, mit Wasser gespült und sodann getrocknet. Das so behandelte Haar ist vom Haaransatz bis zu den Haarspitzen gleichmäßig dunkelbraun gefärbt.

**Beispiel 2: Oxidationshaarfärbemittel**

| Cremeförmige Farbträgermasse (A2) | |
|---|---|
| 21,00 g | Cetylstearylalkohol |
| 4,75 g | Glycerinmonodistearat, selbstemulgierend |

(fortgesetzt)

| Cremeförmige Farbträgermasse (A2) | |
|---|---|
| 4,00 g | Natriumlaurylalkoholdiglykolethersulfat, 28-prozentige wäßrige Lösung |
| 2,00 g | Polyethylenglykol(25)cetyl/stearylether (Cremophor® A25 der BASF AG; Ludwigshafen/DE) |
| 0,70 g | Cholesterin |
| 0,30 g | Resorcin |
| 0,30 g | p-Phenylendiamin |
| 0,20 g | Ascorbinsäure |
| 5,00 g | Ammoniak, 25-prozentige wäßrige Lösung |
| 61,75 g | Wasser |
| $\overline{100,00\ g}$ | |

| Wasserstoffperoxid-Zubereitung (B2) | |
|---|---|
| 12,0 g | Wasserstoffperoxid, 50-prozentige wäßrige Lösung |
| 4,0 g | Polyoxyethylen(45)polyoxypropylen(33)butylether (Unilube® MB50-168 R der Nippon Oil & Fats Co., Ltd.; Tokyo/Japan) |
| 84,0 g | Wasser |
| $\overline{100,0\ g}$ | |

Der pH-Wert der Wasserstoffperoxid-Zubereitung (B2) wird mit verdünnter Phosphorsäure auf 2,5 eingestellt.

In einer Auftrageflasche werden 50 Gramm der Farbträgermasse (A2) und 50 Gramm der Wasserstoffperoxidzubereitung (B2) miteinander vermischt. Nach kurzem Schütteln wird eine vollkommen homogene Mischung erhalten, die nahezu restlos aus der Auftrageflasche entnommen werden kann.

Das so erhaltene Haarfärbemittel wird mit der Auftrageflasche auf ergrautes Haar aufgetragen. Nach einer Einwirkungszeit von 30 Minuten bei Raumtemperatur wird das Haarfärbemittel mit Wasser gründlich ausgespült. Anschließend wird das Haar mit einem Shampoo gewaschen, mit Wasser gespült und sodann getrocknet.

Das so behandelte Haar ist gleichmäßig dunkelblond gefärbt.

**Beispiel 3: Vergleichsversuche**

**Versuch I:**

Ein Oxidationshaarfärbemittel gemäß Beispiel 1 bestehend aus den Komponenten (A1) und (B1) wird bezüglich seiner färberischen Eigenschaften sowie seiner Viskosität, der Mischbarkeit der beiden Komponenten und seiner Applizierbarkeit mittels einer Auftrageflasche mit einem Oxidationshaarfärbemittel verglichen, welches durch Vermischen der Komponente (A1) mit der Komponente (C) erhalten wird. Die Komponente (C) unterscheidet sich hierbei von der Komponente (B1) nur dadurch, daß bei ihr der Polyoxyethylen(45)polyoxypropylen(33)butylether mengengleich durch Wasser ersetzt worden ist.

Die Anwendung der Mittel erfolgt in der in Beispiel 1 beschriebenen Weise.

Die Ergebnisse dieses Vergleichsversuches sind in der nachfolgenden Tabelle I zusammengefaßt.

Tabelle I

|  | erfindungsgemäßes Oxidationshaar-farbemittel (A1) + (B1) | nicht-erfindungsgemäßes Oxidationshaarfärbemittel (A1) + (C) |
|---|---|---|
| Färbeergebnis | in beiden Fällen identisch | |
| Mischbarkeit der beiden Komponenten | sehr gut; homogene Mischung wird nach maximal 10 Sekunden erreicht | durch Schütteln nur schwer mischbar, wobei ein vergleichsweise hoher Kraftaufwand erforderlich ist |
| Viskosität | mittel- bis niedrigviskos | hochviskos |
| Applizierbarkeit mittels einer Auftrageflasche | problemloses Auftragen; in der Auftrageflasche verbleibt eine Restmenge von höchstens 15 % bis 18 % | schwieriges Auftragen; in der Auftrageflasche verbleibt eine Restmenge von mehr als 30 % |

**Versuch II**

Ein Oxidationshaarfärbemittel gemäß Beispiel 2 bestehend aus den Komponenten (A2) und (B2) wird bezüglich seiner färberischen Eigenschaften sowie seiner Gebrauchseigenschaften mit einem Oxidationshaarfärbemittel verglichen, welches durch Vermischen der Komponenten (A2) und (D) erhalten wird. Die Komponente (D) unterscheidet sich hierbei von der Komponente (B2) lediglich dadurch, daß bei ihr der Polyoxyethylen(45)polyoxypropylen(33)butylether mengengleich durch Wasser ersetzt wurde.

Die Anwendung der beiden Mittel erfolgt in der in Beispiel 2 beschriebenen Weise.

Das Ergebnis dieses Vergleichsversuches ist in Tabelle II zusammengefaßt.

Tabelle II

|  | erfindungsgemäßes Oxidationshaarfärbemittel (A2) + (B2) | nicht-erfindungsgemäßes Oxidationshaarfärbemittel (A2) + (D) |
|---|---|---|
| Färbeergebnis | in beiden Fällen identisch | |
| Mischbarkeit der beiden Komponenten | sehr leicht mischbar; homogene Mischung wird nach weniger als 10 Sekungen erhalten | durch Schütteln nur schwer mischbar |
|  | erfindungsgemäßes Oxidationshaarfärbemittel (A1) + (B1) | nicht-erfindungsgemäßes Oxidationshaarfärbemittel (A1) + (D) |
| Viskosität | mittel- bis niedrigviskos | hochviskos |
| Applizierbarkeit mittels einer Auftrageflasche | problemloses Auftragen; in der Auftrageflasche verbleibt nur eine geringe Restmenge (maximal 15 %) | schwieriges Auftragen; es verbleibt eine hohe Restmenge (> 30 %) in der Auftrageflasche |

Sämtliche in der vorliegenden Anmeldung genannten Prozentangaben beziehen sich, sofern nichts anderes angegeben wird, auf Gewichtsprozente.

Für die genannten Viskositätsangaben wurde eine Messung der Viskosität mit einer Viskowaage der Firma Haake, Typ VW; Stab 2; Auflagegewicht: 20 Gramm; Meßtemperatur: 20 Grad Celsius, zugrunde gelegt.

**Patentansprüche**

1.    Mittel zum oxidativen Färben von Haaren, welches durch Vermischen einer hochviskosen, cremeförmigen Farbträgermasse (A) mit einer flüssigen oder emulsionsförmigen, ein Oxidationsmittel enthaltenden Zubereitung (B) erhalten wird, dadurch gekennzeichnet, daß die das Oxidationsmittel enthaltende Zubereitung (B) ein nichtionisches Polymer der Formel (I)

$$R\text{-}(O\text{-}CH(CH_3)\text{-}CH_2)_x\text{-}(O\text{-}CH_2\text{-}CH_2)_y\text{-}OH \qquad (I),$$

mit

R = $C_1$-bis $C_{25}$-Alkyl, $C_2$- bis $C_{25}$-Hydroxyalkyl oder $C_3$- bis $C_{25}$-Dihydroxyalkyl;
x = einer ganzen Zahl von 0 bis 200;
y = einer ganzen Zahl von 0 bis 200 und
   $x + y \geq 20$,

enthält.

2. Mittel nach Anspruch 1, dadurch gekennzeichnet, daß das Gewichtsverhältnis der Färbemasse (A) zur Zubereitung (B) 2:1 bis 1:3 beträgt.

3. Mittel nach Anspruch 1 oder 2, dadurch gekennzeichnet, daß das nichtionische Polymer der Formel (I) ausgewählt ist aus Polyoxyethylen(45)polyoxypropylen(33)butylether, Polyoxyethylen(37)polyoxypropylen(38)butylether, Polyoxyethylen(10)polyoxypropylen(7)butylether, Polyoxyethylen(30)polyoxypropylen(20)butylether, Polyoxyethylen(20)polyoxypropylen(10)cetyl/stearylether, Polyoxyethylen(20)polyoxypropylen(5)cetylether, Polyoxyethylen-(10)polyoxypropylen(20)decyltetradecylether, Polyoxyethylen(24)polyoxypropylen(24)glycerylether, Polyoxyethylen(50)polyoxypropylen(12)lanolinether und Polyoxyethylen(12)polyoxypropylen(66)glycerylether.

4. Mittel nach einem der Ansprüche 1 bis 3, dadurch gekennzeichnet, daß das Polymer der Formel (I) in der Zubereitung (B) in einer Menge von 0,01 bis 6 Gewichtsprozent enthalten ist.

5. Mittel nach einem der Ansprüche 1 bis 4, dadurch gekennzeichnet, daß die Farbträgermasse (A) 15 bis 60 Gewichtsprozent eines Verdickergemisches enthält.

6. Mittel nach Anspruch 5, dadurch gekennzeichnet, daß das Verdickergemisch zu mindestens 50 Gewichtsprozent aus $C_{10}$- bis $C_{24}$-Fettalkoholen besteht.

7. Mittel nach einem der Ansprüche 1 bis 6, dadurch gekennzeichnet, daß die Farbträgermasse (A) 0,01 bis 12 Gewichtsprozent einer Entwicklersubstanz-Kupplersubstanz-Kombination enthält.

8. Mittel nach einem der Ansprüche 1 bis 7, dadurch gekennzeichnet, daß die Farbträgermasse (A) 0,01 bis 6 Gewichtsprozent direktziehende und mit sich selbst kuppelnde Farbstoffvorstufen enthält.

9. Mittel nach einem der Ansprüche 1 bis 8, dadurch gekennzeichnet, daß die Gesamtmenge aller in der Farbträgermasse enthaltenen Farbstoffe 0,1 bis 14 Gewichtsprozent beträgt.

10. Mittel nach einem der Ansprüche 1 bis 9, dadurch gekennzeichnet, daß die Zubereitung (B) 0,1 bis 20 Gewichtsprozent eines Oxidationsmittels enthält.

11. Mittel nach Anspruch 10, dadurch gekennzeichnet, daß das Oxidationsmittel Wasserstoffperoxid ist.

12. Verfahren zum oxidativen Färben von Haaren, dadurch gekennzeichnet, daß man ein Haarfärbemittel nach einem der Ansprüche 1 bis 11 herstellt, indem man unmittelbar vor dem Gebrauch die Farbträgermasse (A) mit der das Oxidationsmittel enthaltenden Zubereitung (B) in einem Verhältnis von 2:1 bis 1:3 vermischt, sodann eine für die Haarfärbung ausreichende Menge des gebrauchsfertigen Oxidationshaarfärbemittels mit Hilfe einer Auftrageflasche auf das Haar aufträgt, es dort 10 bis 45 Minuten lang bei einer Temperatur von 15 bis 50 Grad Celsius einwirken läßt, anschließend das Haar mit Wasser spült und sodann trocknet.

## Claims

1. Agent for the oxidative dyeing of hair, which is obtained by mixing a highly viscous cream-form colour carrier composition (A) and a liquid or emulsion-form preparation (B) containing an oxidising agent, characterised in that the preparation (B) containing the oxidising agent contains a non-ionic polymer of the formula (I)

$$R\text{-}(O\text{-}CH(CH_3)\text{-}CH_2)_x\text{-}(O\text{-}CH_2\text{-}CH_2)_y\text{-}OH \qquad (I),$$

wherein

R = $C_1$-$C_{25}$-alkyl, $C_2$-$C_{25}$-hydroxyalkyl or $C_3$-$C_{25}$-dihydroxyalkyl;
x = an integer from 0 to 200;
y = an integer from 0 to 200; and
$x + y \geqq 20$ .

2. Agent according to Claim 1, characterised in that the ratio by weight of dyeing composition (A) to preparation (B) is from 2:1 to 1:3.

3. Agent according to Claim 1 or 2, characterised in that the non-ionic polymer of the formula (I) is selected from polyoxyethylene(45)polyoxypropylene(33) butyl ether, polyoxyethylene(37)polyoxypropylene(38) butyl ether, polyoxyethylene(10)polyoxypropylene(7) butyl ether, polyoxyethylene(30)polyoxypropylene(20) butyl ether, polyoxyethylene(20)polyoxypropylene(10) cetyl/stearyl ether, polyoxyethylene(20)polyoxypropylene(5) cetyl ether, polyoxyethylene(10)polyoxypropylene(20) decyl tetradecyl ether, polyoxyethylene(24)polyoxypropylene(24) glyceryl ether, polyoxyethylene(50)polyoxypropylene(12) lanoline ether and polyoxyethylene(12)polyoxypropylene(66) glyceryl ether.

4. Agent according to any one of Claims 1 to 3, characterised in that the amount of polymer of the formula (I) contained in preparation (B) is from 0.01 to 6% by weight.

5. Agent according to any one of Claims 1 to 4, characterised in that the colour carrier composition (A) contains from 15 to 60% by weight of a thickener mixture.

6. Agent according to Claim 5, characterised in that at least 50% by weight of the thickener mixture consists of $C_{10}$-$C_{24}$-fatty alcohols.

7. Agent according to any one of Claims 1 to 6, characterised in that the colour carrier composition (A) contains from 0.01 to 12% by weight of a developer substance/coupler substance combination.

8. Agent according to any one of Claims 1 to 7, characterised in that the colour carrier composition (A) contains from 0.01 to 6% by weight of direct and self-coupling dyestuff precursors.

9. Agent according to any one of Claims 1 to 8, characterised in that the total amount of all the dyestuffs contained in the colour carrier composition is from 0.1 to 14% by weight.

10. Agent according to any one of Claims 1 to 9, characterised in that preparation (B) contains from 0.1 to 20% by weight of an oxidising agent.

11. Agent according to Claim 10, characterised in that the oxidising agent is hydrogen peroxide.

12. Process for the oxidative dyeing of hair, characterised in that a hair dyeing agent according to any one of Claims 1 to 11 is prepared by mixing the colour carrier composition (A) and the preparation (B) containing the oxidising agent in a ratio of from 2:1 to 1:3 immediately prior to use, an amount of the ready-for-use oxidising hair dyeing agent sufficient to dye the hair is then applied to the hair by means of an applicator bottle, it is left to take effect there for from 10 to 45 minutes at a temperature of from 15 to 50°C, and then the hair is rinsed with water and subsequently dried.

**Revendications**

1. Agent pour la coloration oxydante des cheveux, qui est obtenu par mélangeage d'une matière colorante à haute viscosité sous forme de crème porteuse (A) avec une préparation (B) liquide ou sous forme d'émulsion contenant un agent d'oxydation liquide, caractérisé en ce que la préparation (B) contenant l'agent d'oxydation contient un polymère non ionique de formule (I)

$$R-(O-CH(CH3)-CH2)x-(O-CH2-CH2)y-OH \qquad\qquad (I)$$

dans laquelle

R = un alkyle en C1-C25, un hydroxyalkyle en C2-C25, ou un dihydroxyalkyle en C3-C25.

x =       un nombre entier allant de 0 à 200.

y =       un nombre entier allant de 0 à 200.
          x + y ≥ 20

2. Agent selon la revendication 1, caractérise en ce que le rapport pondéral de la matière colorante (A) à la préparation (B) va de 2 : 1 à 1 : 3.

3. Agent sel on la revendication 1, caractérisé en ce que le polymère non ionique de formule (I) est choisi parmi

      le polyoxyéthylène(45)polyoxypropylène(33)butyléther,

      le polyoxyéthylène(37)polyoxypropylène(38)butyléther,

      le polyoxyéthylène(10)polyoxypropylène(7)butyléther,

      le polyoxyéthylène(30)polyoxypropylène(20)butylèther,

      le polyoxyéthylène(20)polyoxypropylène(10)cétylstéaryléther,

      le polyoxyéthylène(20)polyoxypropylène(5)cétyléther,

      le polyoxyéthylène(10)polyoxypropylène(20)décyltradécyléther,

      le polyoxyéthylène(24)polyoxypropylène(24)glycéryléther,

      le polyoxyéthylène(50)polyoxypropylène(12)lanolinéther, et

      le polyoxyéthylène(12)polyoxypropylène(66)glycéryléther.

4. Agent selon l'une des revendications 1 à 3, caractérisé en ce que le polymère de formule (I) est contenu dans la préparation (B) à raison de 0,01 à 6% en poids.

5. Agent selon l'une des revendications 1 à 4, caractérisé en ce que la matière colorante (A) contient de 15 à 60% en poids d'un mélange épaississant.

6. Agent sel on la revendication 5 caractérisé en ce que l'agent épaississant est composé au moins 50% en poids d'alcools gras en C10 à C24.

7. Agent selon l'une des revendications 1 à 6, caractérisé en ce que la matière colorante (A) contient de 0,01 à 12% en poids d'une combinaison agent de développement-copulant.

8. Agent selon l'une des revendications 1 à 7, caractérisé en ce que la matière colorante (A) contient de 0,01 à 6% en poids de précurseurs de colorant à fixation directe et auto-copulant.

9. Agent selon l'une des revendications 1 à 8, caractérisé en ce que la quantité totale de tous les colorants contenus dans la matière colorante (B) va de 0,1 à 14% en poids.

10. Agent selon, l'une des revendications 1 à 9, caractérisé en ce que la préparation (B) contient de 0,1 à 20% en poids d'un agent d'oxydation.

11. Agent selon la revendication 10, caractérisé en ce que l'agent d'oxydation est le peroxyde d'hydrogène.

12. Procédé de coloration oxydante des cheveux, caractérisé en ce qu'on prépare une teinture capillaire sel on l'une des revendications 1 à 11 selon lequel, directement avant utilisation, on mélange la matière colorante (A) avec la préparation (B) contenant l'agent d'oxydation, dans un rapport de 2 : 1 à 1 : 3, puis, on apporte sur les cheveux une quantité suffisante pour la teinture de l'agent de coloration capillaire à l'aide d'un flacon d'application, puis on laisse agir de 10 à 45 minutes à une température de 15 à 50°C, on rince alors à l'eau et ensuite on sèche.